# EUROPEAN PATENT APPLICATION

(11) **EP 4 360 694 A1**
(43) Date of publication of application: **01.05.2024**
(21) Application number: 23203218.5
(22) Date of filing: 12.10.2023
(51) Int. Cl.: A61N 1/36, A61B 5/0205, A61B 5/024, A61N 1/05

(54) **DETECTING SIGNALS WITH CARDIAC ACTIVITY DURING DEFINED REST STATES USING NEUROMODULATION SYSTEM**

(30) Priority: 25.10.2022 US 202263419177 P; 22.09.2023 US 202318371893
(71) Applicant: Medtronic, Inc., Minneapolis, MN 55432 (US)
(72) Inventor: Straka, Malgorzata Maria, Blaine (US); Brinda, Annemarie K., Minneapolis (US); Hincapie, Juan G., Minneapolis (US); Litvak, Leonid M., Los Angeles (US); Mueller, Jerel Keith, Minneapolis (US); Nedrud, Joshua James, Minneapolis (US); Cleland, Andrew Jay, Minneapolis (US); Kharam, Aleksandra Pavlovna, Minneapolis (US)
(74) Representative: Zimmermann & Partner Patentanwälte mbB

(57) **Abstract**

A system is provided for informing a therapeutic procedure. In some embodiments, the system may be configured to determine specific states associated with a sleep-wake cycle of a patient. Additionally, the system may be configured to measure one or more signals with cardiac activity of the patient upon detecting the specific states of the sleep-wake cycle of the patient. Subsequently, the system may be configured to determine one or more parameters for applying an electrical signal to an anatomical element of the patient based on the one or more signals with cardiac activity. In some embodiments, the system may be configured to establish a longitudinal trend of cardiac metrics for the patient based on a plurality of signals with cardiac activity for the patient measured during the specific states of the sleep-wake cycle across a plurality of days.

## Description

### CROSS-REFERENCE TO RELATED APPLICATIONS

This application claims the benefit of U.S. Provisional Application No. 63/419,177, filed on October 25, 2022, which application is incorporated herein by reference in its entirety.

### BACKGROUND

The present disclosure is generally directed to therapeutic neuromodulation, and relates more particularly to detecting signals with cardiac activity for supporting the therapeutic neuromodulation.

Neuromodulation therapy may be carried out by sending an electrical signal generated by a device (e.g., a pulse generator) to a stimulation target (e.g., nerves, non-neuronal cells, etc.), which may provide a desired electrophysiologic, biochemical, or genetic response in the stimulation target. Neuromodulation therapy systems may be used to deliver electrical stimulation for providing chronic pain treatment to a patient. In some neuromodulation therapies (e.g., closed-loop neuromodulation therapies), one or more signals resulting from the neuromodulation may be recorded and the therapy may be adjusted based on the recorded signals.

### BRIEF SUMMARY

Example aspects of the present disclosure include:
A system for informing a therapeutic procedure, comprising: a pulse generator configured to generate a therapeutic electrical signal; one or more leads in communication with the pulse generator and configured to transmit the therapeutic electrical signal to a plurality of electrodes; the plurality of electrodes in communication with the one or more leads, the plurality of electrodes configured to apply the therapeutic electrical signal to an anatomical element of the patient and configured to measure a physiological response; a processor; and a memory storing data for processing by the processor. In some embodiments, the data, when processed, may cause the processor to: determine specific states associated with a sleep-wake cycle of a patient; measure, via one or more of the plurality of electrodes, one or more signals with cardiac activity of the patient upon detecting the specific states of the sleep-wake cycle of the patient; and determine one or more parameters for applying the therapeutic electrical signal to the anatomical element based at least in part on the one or more signals with cardiac activity.

Any of the aspects herein, wherein the memory stores further data for processing by the processor that, when processed, causes the processor to: establish a longitudinal trend of cardiac metrics for the patient based at least in part on a plurality of signals with cardiac activity for the patient measured during the specific states of the sleep-wake cycle across a plurality of days.

Any of the aspects herein, wherein the memory stores further data for processing by the processor that, when processed, causes the processor to: determine one or more cardiac metrics fall outside the longitudinal trend of cardiac metrics; and adjust one or more therapy parameters for applying the therapeutic electrical signal to the anatomical element based at least in part on determining the one or more cardiac metrics fall outside the longitudinal trend of cardiac metrics, or provide, via a user interface, an alert indicating that the one or more cardiac metrics fall outside the longitudinal trend of cardiac metrics.

Any of the aspects herein, wherein the one or more adjusted therapy parameters comprise an amplitude adjustment, adjustment of frequency, adjustment of pulse width, adjustment of duty cycling, adjustment of cycling of high frequency or low frequency components independently, adjustment of charge balancing strategy, adjustment of selection of the plurality of electrodes, or a combination thereof.

Any of the aspects herein, wherein the memory stores further data for processing by the processor that, when processed, causes the processor to: output, via a user interface, the longitudinal trend of cardiac metrics for the patient, wherein the one or more parameters for applying the therapeutic electrical signal to the anatomical element are determined based at least in part on outputting the longitudinal trend of cardiac metrics for the patient.

Any of the aspects herein, wherein the data stored in the memory that, when processed causes the processor to measure the one or more signals with cardiac activity further causes the system to: measure, via one or more of the plurality of electrodes, a first signal with cardiac activity during a first specific state of the sleep-wake cycle; and measure, via one or more of the plurality of electrodes, a second signal with cardiac activity during a second specific state of the sleep-wake cycle, wherein the one or more parameters for applying the therapeutic electrical signal to the anatomical element are determined based at least in part on a comparison of the first signal and the second signal.

Any of the aspects herein, wherein the first specific state comprises a first sleep state for the patient, and the second specific state comprises a second sleep state for the patient.

Any of the aspects herein, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.

Any of the aspects herein, wherein the memory stores further data for processing by the processor that, when processed, causes the processor to: configure a duration for measuring the one or more signals with cardiac activity, wherein the one or more signals with cardiac activity are measured according to the configured duration.

Any of the aspects herein, wherein the specific states of the sleep-wake cycle are detected based at least in part on an algorithm that uses accelerometry measurements, cardiac metrics, a time of day, respiration measurements, additional information from external devices, or a combination thereof, as input parameters.

Any of the aspects herein, wherein the signals with cardiac activity comprise cardiac electrogram measurements, and wherein one or more cardiac metrics are derived from the cardiac electrogram measurements, the one or more cardiac metrics comprising a heart rate, a heart rate variability, respiration, or a combination thereof, for the patient.

A system for informing a therapeutic procedure, comprising: a processor; and a memory storing data for processing by the processor, the data, when processed, causes the processor to: determine specific states associated with a sleep-wake cycle of a patient; measure one or more signals with cardiac activity of the patient upon detecting the specific states of the sleep-wake cycle of the patient; and determine one or more parameters for applying a therapeutic electrical signal to an anatomical element of the patient based at least in part on the one or more signals with cardiac activity.

Any of the aspects herein, wherein the memory stores further data for processing by the processor that, when processed, causes the processor to: establish a longitudinal trend of cardiac metrics for the patient based at least in part on a plurality of signals with cardiac activity for the patient measured during the specific states of the sleep-wake cycle across a plurality of days.

Any of the aspects herein, wherein the memory stores further data for processing by the processor that, when processed, causes the processor to: determine one or more cardiac metrics fall outside the longitudinal trend of cardiac metrics; and adjust one or more therapy parameters for applying the therapeutic electrical signal to the anatomical element based at least in part on determining the one or more cardiac metrics fall outside the longitudinal trend of cardiac metrics, or provide, via a user interface, an alert indicating that the one or more cardiac metrics fall outside the longitudinal trend of cardiac metrics.

Any of the aspects herein, wherein the one or more adjusted therapy parameters comprise an amplitude adjustment, frequency adjustment, pulse width adjustment, adjustment of duty cycling, adjustment of cycling of high frequency or low frequency components independently, adjustment of charge balancing strategy, adjustment of selection of the plurality of electrodes, or a combination thereof.

Any of the aspects herein, wherein the data stored in the memory that, when processed causes the processor to measure the one or more signals with cardiac activity further causes the system to: measure a first signal with cardiac activity during a first specific state of the sleep-wake cycle; and measure a second signal with cardiac activity during a second specific state of the sleep-wake cycle, wherein the one or more parameters for applying the therapeutic electrical signal to the anatomical element are determined based at least in part on a comparison of the first signal and the second signal.

Any of the aspects herein, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.

Any of the aspects herein, wherein the memory stores further data for processing by the processor that, when processed, causes the processor to: configure a duration for measuring the one or more signals with cardiac activity, wherein the one or more signals with cardiac activity are measured according to the configured duration.

Any of the aspects herein, wherein the specific states of the sleep-wake cycle are detected based at least in part on an algorithm that uses accelerometry measurements, cardiac metrics, a time of day, respiration measurements, additional information from external devices, or a combination thereof, as input parameters.

A system for informing a therapeutic procedure, comprising: a pulse generator configured to generate a therapeutic electrical signal; one or more leads in communication with the pulse generator and configured to transmit the therapeutic electrical signal to a plurality of electrodes, the plurality of electrodes disposed at a distal end of the one or more leads; and the plurality of electrodes in communication with the one or more leads and configured to be implanted near a stimulation target, the plurality of electrodes configured to apply the therapeutic electrical signal to an anatomical element of the patient and configured to measure a physiological response, wherein the therapeutic electrical signal is applied to the anatomical element based at least in part on one or more signals with cardiac activity measured via one or more of the plurality of electrodes at specific states of a sleep-wake cycle of the patient.

Any of the aspects herein, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.

Any aspect in combination with any one or more other aspects.

Any one or more of the features disclosed herein.

Any one or more of the features as substantially disclosed herein.

Any one or more of the features as substantially disclosed herein in combination with any one or more other features as substantially disclosed herein.

Any one of the aspects/features/embodiments in combination with any one or more other aspects/features/embodiments.

Use of any one or more of the aspects or features as disclosed herein.

Further disclosed herein is a system for informing a therapeutic procedure, wherein the system may be configured to determine specific states associated with a sleep-wake cycle of a patient, wherein, additionally, the system may be configured to measure one or more signals with cardiac activity of the patient upon detecting the specific states of the sleep-wake cycle of the patient, wherein, subsequently, the system may be configured to determine one or more parameters for applying an electrical signal to an anatomical element of the patient based on the one or more signals with cardiac activity, wherein, in some embodiments, the system may be configured to establish a longitudinal trend of cardiac metrics for the patient based on a plurality of signals with cardiac activity for the patient measured during the specific states of the sleep-wake cycle across a plurality of days.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present disclosure will become apparent to those skilled in the art upon consideration of the embodiment descriptions provided hereinbelow.

### BRIEF DESCRIPTION OF THE SEVERAL VIEWS OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2 is an example cardiac signal measurement according to at least one embodiment of the present disclosure;
Fig. 3 is an example rest state tracking according to at least one embodiment of the present disclosure;
Fig. 4 is an example of long term metrics according to at least one embodiment of the present disclosure;
Fig. 5 is a diagram of a system according to at least one embodiment of the present disclosure;
Fig. 6 is a flowchart of a method according to at least one embodiment of the present disclosure;
Fig. 7 is a flowchart of a method according to at least one embodiment of the present disclosure; and
Fig. 8 is a flowchart of a method according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., random-access memory (RAM), read-only memory (ROM), electrically erasable programmable ROM (EEPROM), flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Cortex Mx; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to a device, operator, or user of the system, and distal being further from the device, operator, or user of the system.

For some closed-loop neuromodulation therapies, a therapeutic electrical signal generated by a pulse generator may be sent to a stimulation target (e.g., nerves, non-neuronal cells, etc.). A biopotential (e.g., recorded signal) elicited with the therapeutic electrical signal may be recorded. The elicited biopotential may provide information by which to adjust the therapeutic electrical signal. Other types of closed-loop neuromodulation therapies may use and sense other types of signals to determine adjustments for the therapeutic electrical signal, such as outputs of other sensors implanted in or placed on a patient (e.g., posture sensor, accelerometer, etc.).

In some examples, spinal cord stimulation (SCS) (e.g., a form of neuromodulation that includes applying a therapeutic electrical signal or stimulation signal to nerves of the spinal cord or nerves near the spinal cord to elicit a desired electrophysiologic, biochemical, or genetic response) may be practiced in a closed-loop manner. When SCS is performed in a closed-loop manner, contacts (e.g., leads, electrodes, etc.) may be placed near a stimulation target (e.g., patient's spinal cord or a proximate structure (such as the dorsal root ganglion) or one or more targets), where the contacts are configured to apply a therapeutic electrical signal to the stimulation target to obtain a desirable electrophysiologic, biochemical, or genetic state (e.g., that leads to pain relief). For example, the therapeutic electrical signal may be configured to change how the patient's body interprets a pain signal based on causing a desired electrophysiologic, biochemical, or genetic response when applied to the stimulation target.

While SCS neuromodulation or neurostimulation systems are typically used to deliver electrical stimulation for chronic pain, physiological recordings from the SCS system can provide key information about the health state of the patient. For example, cardiac electrogram signals of high quality can be recorded from implanted SCS leads configured to apply the therapeutic electrical signal to the nerves and/or spinal cord of the patient.

Cardiac electrogram signals can be combined with accelerometry signals to provide a host of capabilities to the SCS system in addition to providing stimulation for pain treatment. Cardiac monitoring over time is an important capability. Cardiac metrics (e.g., derived from the cardiac electrogram signals and/or additional signals), such as heart rate and heart rate variability (HRV), are susceptible to daily activities and stressors. Therefore, to establish a longitudinal trend of cardiac metrics for a patient, measurements need to be taken during similar conditions each day. In some cases, an HRV measurement taken by the patient upon waking from sleep may be optimal as there is minimal possibility of stress to impact the HRV measurement at that instance. Calculated HRV metrics may include (but are not limited to) time series metrics, such as standard deviation N-N (SDNN) intervals of normal heart beats and the root mean squared of successive differences (RMSSD) between normal heart beats, frequency domain metrics, such as high-frequency and low frequency HRV, and time-frequency metrics. These HRV metrics provide different insights into autonomic nervous system function. For example, RMSSD and high frequency HRV are associated with parasympathetic activity.

Previous techniques have been developed for recording signals that indicate cardiac activity from or on SCS leads. For example, the previously developed techniques use far-field cardiac signals sensed by SCS to control stimulation to address cardiovascular disorders. However, the previously developed techniques do not consider finding consistent rest-states using input signals to track metrics to better understand a health state. There is a lot of variability in cardiac activity over the course of the day/night for a patient, such that observing how the health state of the patient changes with time at similar rest states (e.g., both for open-loop monitoring and closed-loop therapy) is beneficial for recording signals that indicate cardiac activity of the patient.

As described herein, an SCS system is provided that is capable of triggering a cardiac measurement without user interaction (e.g., from the patient or a clinician) by detecting a similar resting state each morning before or after waking for the patient to perform the cardiac measurement. In some embodiments, the SCS system may establish a longitudinal trend of a patient's cardiac metrics (e.g., such as heart rate, HRV, and cardiac electrogram-derived respiration) by measuring daily and/or nightly cardiac electrogram signals using the SCS system during a consistent resting state for the patient. The daily and/or nightly measurements may be performed using components of the implantable neuromodulation system with no additional wearables necessary.

Accelerometry alone, and especially in combination with heart rate and heart rate variability, often provides enough information to reliably predict if a person is awake or asleep. Accelerometry and cardiac signals together provide reliable predictions of sleep stages (e.g., Wake, NREM1/2, NREM 3/4, and REM). In one embodiment, an algorithm with cardiac and accelerometry input signals (e.g., among other input signals, such as time of day, respiration measurements, information from external devices, etc.) may be used to detect when the patient is waking and may trigger a cardiac electrogram measurement while the person is waking (e.g., but is still in bed). In another embodiment, an algorithm with cardiac and accelerometry input signals (e.g., among other input signals) may be used to detect sleep stages in order to measure cardiac electrogram signals during a similar sleep stage each night. For example, the measurement could be configured to occur in the last NREM1 stage before waking occurs. Accordingly, the cardiac electrogram measurement (e.g., cardiac metric measurement) may be performed for the patient at a consistent sleep or rest state each night/moming guided by a sleep onset/sleep stage detection algorithm. In some embodiments, the cardiac electrogram measurement could be configured to last from a first duration (e.g., 30 seconds) up to a second duration (e.g., 10 minutes). In another embodiment, the cardiac electrogram measurement could be configured to last the entire duration of the selected state.

After the longitudinal trend of the patient's cardiac metrics has been established, the daily/nightly cardiac electrogram-derived metrics (e.g., heart rate, HRV, respiration, etc.) could be used to inform closed-loop neuromodulation. For example, a decrease in the parasympathetic aspects of HRV (e.g., compared to the longitudinal trend) could indicate that a change in therapy is needed to maintain or increase pain relief for the patient. This therapy adjustment could include, but is not limited to, a change in amplitude (e.g., increase or decrease), adjustment of cycling, adjustment of cycling of high frequency or low frequency components independently, adjustment of charge balancing strategy (e.g., recharge adjustments), adjustment of contacts (e.g., adjusting a selection of leads and/or electrodes configured to apply the electrical stimulation signal, adjusting which leads and/or electrodes apply the electrical stimulation signal, etc.), or a combination thereof. The closed-loop therapy adjustment strategy could be implemented during a trial phase of SCS for the patient or after full implantation of the SCS system within the patient. Additionally or alternatively, the difference in cardiac electrogram-derived metrics collected at two different states may be used to inform closed-loop therapy. For example, the HRV at different sleep states may be compared and used to guide the closed-loop therapy. Additionally or alternatively, the daily/nightly cardiac electrogram-derived metrics (e.g., heart rate, HRV, respiration, etc.) and/or the longitudinal trend may be provided (e.g., displayed via a user interface) to physicians, clinicians, the patient, etc., for analysis, patient monitoring, determining parameters and/or therapy adjustments, or a combination thereof.

Embodiments of the present disclosure beneficially enable measuring cardiac metrics and movements of a patient (e.g., for the purpose of life, sleep, and activity tracking) to inform an implantable neuromodulation or neurostimulation system (e.g., SCS system). Embodiments of the present disclosure also beneficially enable a user-friendly neuromodulation system that performs closed-loop adjustments without the addition of extra devices (e.g., wearables) and/or inputs from the patient. Embodiments of the present disclosure further beneficially enable an improved SCS therapy based on obtaining activity, vital signs, and sleep features of a patient.

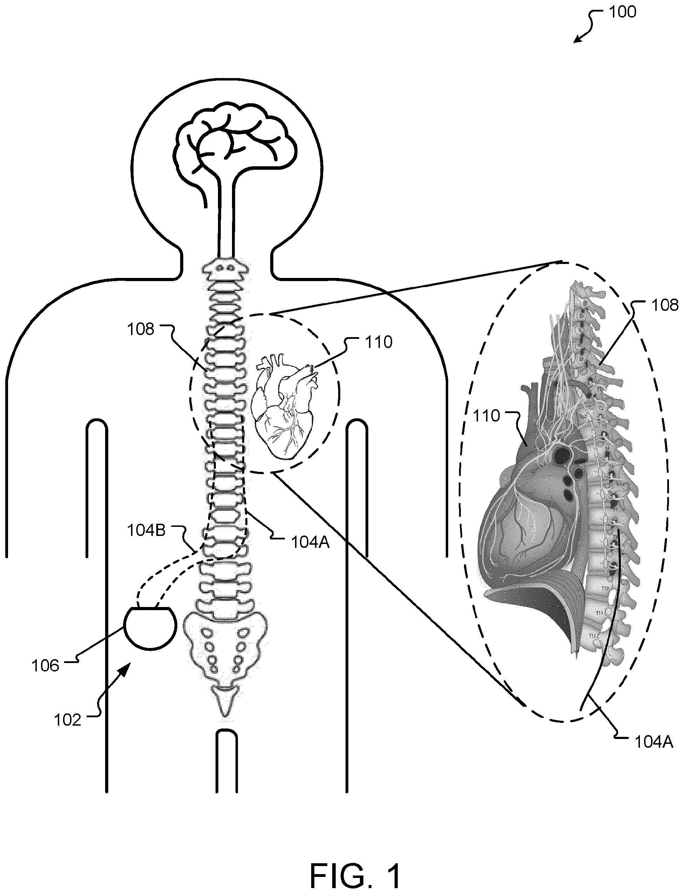

Turning to Fig. 1, a diagram of aspects of a system 100 according to at least one embodiment of the present disclosure is shown. The system 100 may be used to provide electric signals for a patient and/or carry out one or more other aspects of one or more of the methods disclosed herein. For example, the system 100 may include at least a device 102 that is capable of providing a stimulation applied to the spinal cord 108 of the patient and/or to one or more nerve endings for a patient (e.g., for SCS therapy). In some examples, the device 102 may be referred to as an implantable pulse generator 106. More specifically, the implantable pulse generator 106 may be configured to generate a current or therapeutic electrical signal, such as a signal capable of stimulating a response in the spinal cord 108 or from one or more nerves. In some embodiments, as described herein, the device 102 may be implanted within the patient.

Additionally, the system 100 may include one or more leads 104 (e.g., electrical leads) that provide a connection between the device 102 and the spinal cord or nerves of the patient for enabling, for example, stimulation. In some embodiments, the leads 104 may be implanted wholly or partially within the patient.

In some embodiments, the one or more leads 104 may include a first lead 104A disposed on or connected to a first side of the spinal cord 108 of the patient and a second lead 104B disposed on or connected to a second side of the spinal cord 108 of the patient. For example, the first lead 104A may be connected to the righthand side of the spinal cord 108, while the second lead 104B may be connected to the lefthand side of the spinal cord 108. However, the position and/or orientation of each lead relative to the spinal cord 108 may vary depending on, for example, the type of treatment, the type of lead, combinations thereof, and the like. In another example, the first lead 104A and the second lead 104B may overlap one another, and may be placed proximate one another on the dorsal side of the spinal cord 108 close to a midline of the spinal cord 108. In some examples, the first lead 104A and the second lead 104B may both be placed on the midline of the spinal cord 108, where one of the leads 104 is cranial (e.g., anterior or nearer the head of the patient) and the other of the leads 104 is caudal (e.g., posterior or nearer the tail of the patient). Additionally or alternatively, the first lead 104A and the second lead 104B may both be placed on one side of the midline of the spinal cord 108.

Additionally, the one or more leads 104 may be connected, placed, or otherwise implanted near or on the spinal cord 108 within the patient, such that at least one of the one or more leads 104 are located near the heart 110 of the patient. For example, as shown in the inset or zoomed-in portion of the example of Fig. 1 which depicts a side view of the encircled area within the patient, the first lead 104A may be placed within the spinal canal behind the heart 110 (e.g., dorsally within the spinal canal, such as behind a foramen of the spine near the top of a vertebra of the thoracic vertebrae column of the spinal cord 108, or anteriorly within the spinal canal). Additionally or alternatively, as described previously, the exact placement of the one or more leads 104 may vary depending on, for example, the type of treatment, the type of lead, the patient, combinations thereof, and the like. While not specifically shown in the example of Fig. 1, the one or more leads 104 may also exit the spinal cord 108 at a lumbar vertebra lower down the spinal cord 108 (e.g., the L2 vertebra, but the exact location may vary). As described herein, the one or more leads 104 being placed proximate to the heart 110 may enable the system 100 to more effectively capture signals that include cardiac activity before, during, and/or after providing a neuromodulation therapy (e.g., SCS therapy).

In other embodiments, the one or more leads 104 may include at least the first lead 104A and the second lead 104B connected to other nerves of the patient (e.g., the vagus nerve, different trunks of the vagus nerve, etc.). For example, the first lead 104A may be connected to a first nerve (e.g., first vagal trunk of the patient, such as the anterior sub diaphragmatic vagal trunk at the hepatic branching point of the vagus nerve) and the second lead 104B may be connected to a second nerve (e.g., second vagal trunk of the patient, such as the posterior sub diaphragmatic vagal trunk at the celiac branching point of the vagus nerve). The first lead 104A and/or the second lead 104B may be configured to provide an electrical stimulation signal from the device 102 to the respective first and/or second nerve. The connection of the leads 104 to the respective nerve (or other nerves) of the patient may permit the device 102 to measure and/or stimulate one or more evoked potentials (e.g., evoked compound action potentials (ECAPs)) in the patient based on the provided electrical stimulation from the implantable pulse generator 106.

In some examples, the leads 104 may provide the therapeutic electrical signals to the respective nerves via electrodes or electrode devices that are connected to the nerves (e.g., sutured in place, wrapped around the nerves, etc.). In some examples, the leads 104 may be referenced as cuff electrodes or may otherwise include the cuff electrodes (e.g., at an end of the leads 104 not connected or plugged into the device 102). For example, electrodes, electrode devices, cuff electrodes, paddle electrodes, or a different type of electrode may be disposed at a distal end of each of the leads 104.

In other examples, the leads 104 may be or comprise linear SCS leads capable of delivering one or more stimulation signals (e.g., generated by the device 102) to the spinal cord 108. The leads 104 may comprise a plurality of electrodes disposed along the length of the lead, such that the leads 104 contact the spinal cord 108 at multiple points along a length of the spinal cord 108. A first set of the electrodes on each lead may pass a therapeutic electrical signal into the spinal cord 108, while a second set of the electrodes on each lead may sense one or more signals generated in response by the spinal cord 108 (e.g., recorded signals). In one embodiment, the electrodes may be able to sense, measure, or otherwise collected data such as ECAPs. Additionally or alternatively, the electrodes may be able to sense, measure, or otherwise collected data related to cardiac metrics for the patient (e.g., heart rate, HRV, respiration, or other cardiac-derived metrics). In some examples, the device 102 may be used as a contact and/or may include additional contacts for sensing, measuring, or otherwise collecting data related to cardiac metrics for the patient. For example, the heart conducts electrical activity, and depending on the electrode locations, the heart vector can change and different cardiac metrics can be recorded.

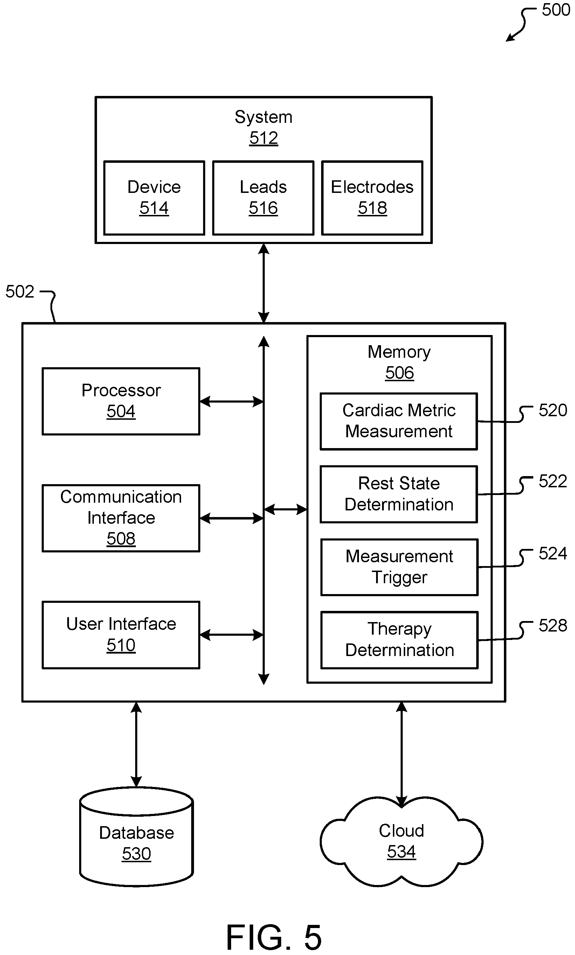

Additionally, while not shown, the system 100 may include one or more processors (e.g., one or more DSPs, general purpose microprocessors, graphics processing units, ASICs, FPGAs, or other equivalent integrated or discrete logic circuitry) shown and described in Fig. 5 that are programmed to carry out one or more aspects of the present disclosure. In some examples, the one or more processors may include a memory or may be otherwise configured to perform the aspects of the present disclosure. For example, the one or more processors may provide instructions to the device 102, the leads 104, the electrodes, or other components of the system 100 not explicitly shown or described with reference to Fig. 1 for applying a stimulation, performing measurements (e.g., cardiac metrics, ECAPs, etc.), and analyzing the same, as described herein. In some examples, the one or more processors may be part of the device 102 or part of a control unit for the system 100 (e.g., where the control unit is in communication with the device 102 and/or other components of the system 100).

As described herein, the system 100 (e.g., an SCS system) may be configured to trigger a cardiac measurement without user interaction (e.g., from the patient or a clinician) by detecting a similar resting state at a same time of the day before or after waking for the patient to perform the cardiac measurement. Additionally, the system 100 may be configured to establish a longitudinal trend of a patient's cardiac metrics (e.g., such as heart rate, HRV, and cardiac-derived respiration) by measuring daily and/or nightly cardiac electrogram signals using the device 102, the leads 104, and/or the electrodes during a consistent resting state for the patient. In some implementations, the longitudinal trend of the patient's cardiac metrics and/or the daily/nightly cardiac electrogram measurements may be reported out (e.g., displayed via a user interface) for analysis, patient monitoring, etc. The daily and/or nightly measurements may be performed using the components of the system 100 with or without additional wearable devices on the patient.

The device 102 may be programmed to measure and record movements of the patient (e.g., for the purpose of life, sleep, and activity tracking). For example, the device 102 may comprise an accelerometer and/or other components that are designed to track and record movements of the patient (e.g., whether the patient is moving, not moving, laying down, standing up, running, walking, etc.). Additionally, the leads 104 and/or electrodes disposed at the distal end of the leads 104 may be programmed to measure a physiological response of the patient. In some examples, the physiological response may comprise an evoked response (e.g., ECAP measurement) based on applying the therapeutic electrical signal (e.g., stimulation signal) generated by the device 102 to the spinal cord 108 (e.g., and/or to nearby nerves as described previously). Additionally or alternatively, as described herein, the physiological signals may comprise cardiac electrogram signals from which cardiac metrics (e.g., heart rate, HRV, respiration, other cardiac-related measurements, etc.) of the patient before and after the therapeutic electrical signal is applied can be derived. In some examples, the device 102 may be programmed to measure and record signals in addition or alternative to the leads 104 and/or electrodes.

Physiological signals (e.g., heart rate, HRV, etc.) may vary during the day and at different sleep cycles at night, and in response to stressors like activity. When evaluating holistic cardiac measures, it is beneficial to compare the measurements at a similar rest state (e.g., or similar sleep state). That is, to establish a longitudinal trend of cardiac metrics for a patient, cardiac measurements need to be taken during similar conditions each day. As an example, an HRV measurement taken by the patient upon waking from sleep may be optimal as there is minimal possibility of stress to impact the HRV measurement at that instance.

In one embodiment, an algorithm with cardiac and accelerometry input signals (e.g., measured and recorded by the device 102, the leads 104, and/or the electrodes as described above) may be used to detect when the patient is waking and may trigger a cardiac electrogram measurement while the person is waking (e.g., but is still in bed). In another embodiment, an algorithm with the cardiac and accelerometry input signals may be used to detect sleep stages in order to measure cardiac electrogram signals during a similar sleep cycle and stage each night for the patient. For example, the measurement could be configured to occur in the last NREM1 stage before waking occurs. Accordingly, the cardiac electrogram measurement (e.g., cardiac metric measurement) may be performed for the patient at a consistent sleep state each night/morning guided by a sleep onset/sleep stage detection algorithm. In some embodiments, the cardiac electrogram measurement could be configured to last from a first duration (e.g., 30 seconds) up to a second duration (e.g., 10 minutes).

That is, the algorithm described and provided herein may identify appropriate timepoints for the system 100 to collect relevant data (e.g., the cardiac metrics, cardiac electrogram measurements, etc.). In some embodiments, the appropriate timepoints may include particular sleep stage(s). Additionally or alternatively, the cardiac metrics may be measured over the course of the entire night, but may be weighted towards specific sleep stage(s). In some embodiments, the appropriate timepoints may include the instances as the patient wakes up. In some examples, the algorithm may use additional input signals (e.g., in addition to the cardiac and accelerometry input signals), such as signals from other sensors and/or information (e.g., time of day, respiration measurements, etc.), as well as information that could be external to the system 100, such as information from a smartphone, a wearable device, etc. For example, the additional input signals may be input to the algorithm based on a synchronization schedule (e.g., weekly, monthly, etc.) with external devices to get more refined inputs for the algorithm.

After the longitudinal trend of the patient's cardiac metrics has been established, the daily/nightly cardiac electrogram-derived metrics (e.g., heart rate, HRV, respiration, etc.) could be used to inform closed-loop neuromodulation. For example, a decrease in HRV (e.g., compared to the longitudinal trend) could indicate that a change in therapy is needed to maintain or increase pain relief for the patient. This therapy adjustment could include, but is not limited to, a change in amplitude (e.g., increase or decrease a strength of the therapeutic electrical signal generated by the device 102), adjustment of cycling of the therapeutic electrical signal (e.g., how long the therapeutic electrical signal is applied, how often the therapeutic electrical signal is applied, etc.), adjustment of frequency of stimulation, adjustment of pulse width, adjustment of cycling of high frequency or low frequency components independently, adjustment of charge balancing strategy (e.g., how charge balance is achieved, such as making recharge adjustments between active recharge, passive recharge, a combination of active and passive recharge, or any type of recharge), adjustment of contacts (e.g., adjusting a selection of the leads 104 and/or electrodes configured to apply the electrical stimulation signal, adjusting which leads 104 and/or electrodes apply the electrical stimulation signal, etc.). In some examples, a physician, clinician, operator, etc. may provide limits as to any of the therapy adjustments. The closed-loop therapy adjustment strategy could be implemented during a trial phase of SCS for the patient or after full implantation of the SCS system within the patient. Additionally or alternatively, the difference in cardiac electrogram-derived metrics (e.g., cardiac metrics) collected at two different states may be used to inform closed-loop therapy. For example, the HRV at different sleep states may be compared and used to guide the closed-loop therapy.

Additionally or alternatively, the longitudinal trend of the patient's cardiac metrics and/or the daily/nightly cardiac electrogram-derived metrics may be provided (e.g., displayed via a user interface) to a physician, a clinician, an operator, the patient, or another user to track overall patient health (e.g., for patient monitoring). In some embodiments, the longitudinal trend of the patient's cardiac metrics and/or the daily/nightly cardiac electrogram-derived metrics may be provided to the physician, clinician, operator, patient, or other user for review and/or analysis to inform or determine therapy adjustment decisions. Additionally or alternatively, the longitudinal trend of the patient's cardiac metrics and/or the daily/nightly cardiac electrogram-derived metrics may be provided to an additional system or device associated with the patient.

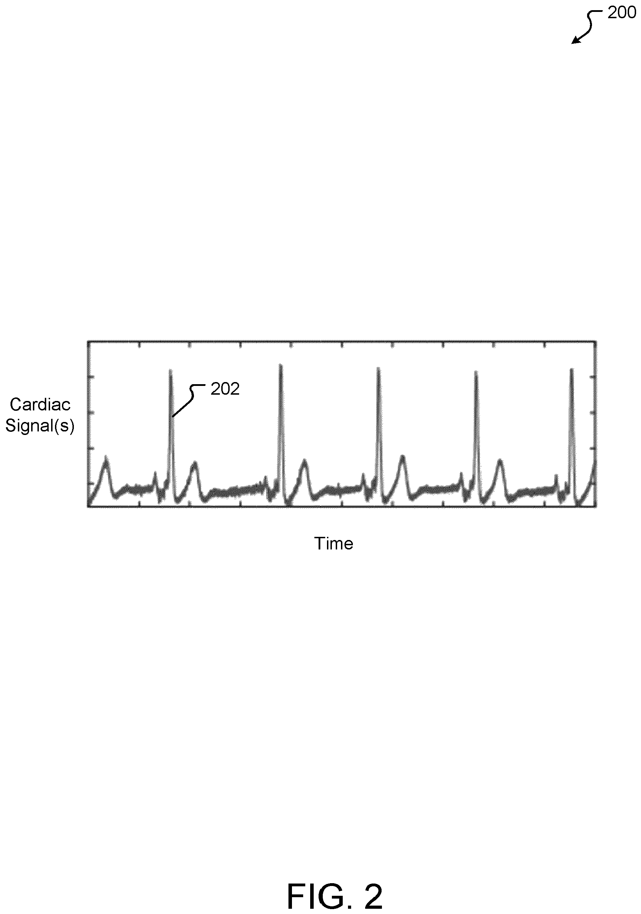

Fig. 2 is an example cardiac signal measurement 200 according to at least one embodiment of the present disclosure. The example cardiac signal measurement 200 may be acquired by one or more aspects of Fig. 1. For example, the example cardiac signal measurement 200 may represent or include a raw cardiac signal 202 recorded from SCS leads that can be used for cardiac electrogram analysis, where the SCS leads may be an example of the leads 104 and/or electrodes as described with reference to Fig. 1. As described previously, the raw cardiac signal 202 may be combined with accelerometry signals (e.g., captured by the device 102, leads 104, and/or electrodes) to be used as inputs for an algorithm to determine appropriate times (e.g., detect a consistent resting state, such as when a patient is waking and/or specific sleep states) for triggering a cardiac metric measurement. The cardiac metric measurement taken across multiple days at the same appropriate time(s) may then be used to develop long term trends for the patient (e.g., longitudinal trend of the patient's cardiac metrics), which can be used to inform closed-loop neuromodulation as described herein.

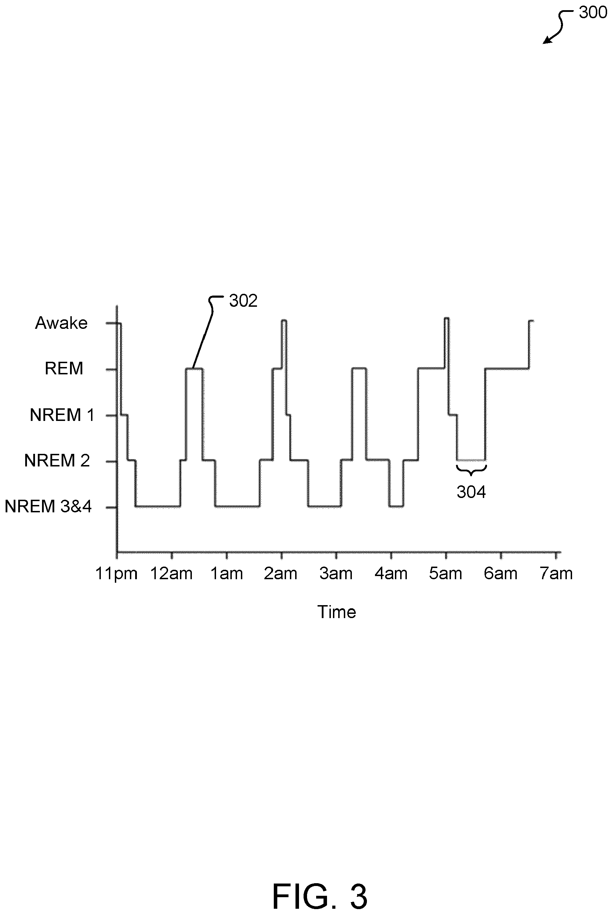

Fig. 3 is an example rest state tracking 300 according to at least one embodiment of the present disclosure. The example rest state tracking 300 may implement aspects of or may be implemented by aspects of Fig. 1. For example, the example rest state tracking 300 may include a sleep-wake cycle 302 that is captured and/or tracked for a patient, such as by the device 102, the leads 104, and/or the electrodes as described with reference to Fig. 1. In some embodiments, the sleep-wake cycle 302 tracked for the patient may enable cardiac measurements to be performed at a consistent rest state for the patient. The sleep-wake cycle described herein refers to the 24-hour cycle to include states of wakefulness and states of rest or sleep. In some embodiments, the rest state chosen for cardiac measurements could be wakeful-rest or sleeping-rest. In some embodiments, the rest-state could specifically be during sleep. And in other embodiments, the rest-state could be a specific sleep stage during sleep. For example, a rest state 304 may be determined for measuring the cardiac metrics described herein at a consistent time for the patient. In the example of Fig. 3, the rest state 304 may represent an NREM 2 sleep state for the patient. Additionally or alternatively, other sleep states may be used for triggering the cardiac metric measurement.

As described previously with reference to Fig. 1, an algorithm may be provided and used herein for determining and/or detecting the rest state 304 for then triggering the cardiac metric measurement. For example, the algorithm may use cardiac and accelerometry input signals (e.g., measured and recorded by the device 102, the leads 104, and/or the electrodes as described above) to detect when the patient is waking and may trigger the cardiac measurement (e.g., cardiac electrogram measurement) while the person is waking (e.g., but is still in bed). In another embodiment, the algorithm (e.g., or a different algorithm) may use cardiac and accelerometry input signals to detect sleep stages in order to capture the cardiac measurement during a similar sleep cycle and stage each night for the patient. That is, the algorithm may identify appropriate timepoints for components of the system 100 to collect relevant data (e.g., the cardiac metrics, cardiac electrogram measurements, etc.). In some examples, the algorithm may use additional input signals (e.g., in addition to the cardiac and accelerometry input signals), such as signals from other sensors and/or information (e.g., time of day, respiration measurements, etc.), as well as information that could be external to the system 100, such as information from a smartphone, a wearable device, etc. For example, the additional input signals may be input to the algorithm based on a synchronization schedule (e.g., weekly, monthly, etc.) with external devices to get more refined inputs for the algorithm.

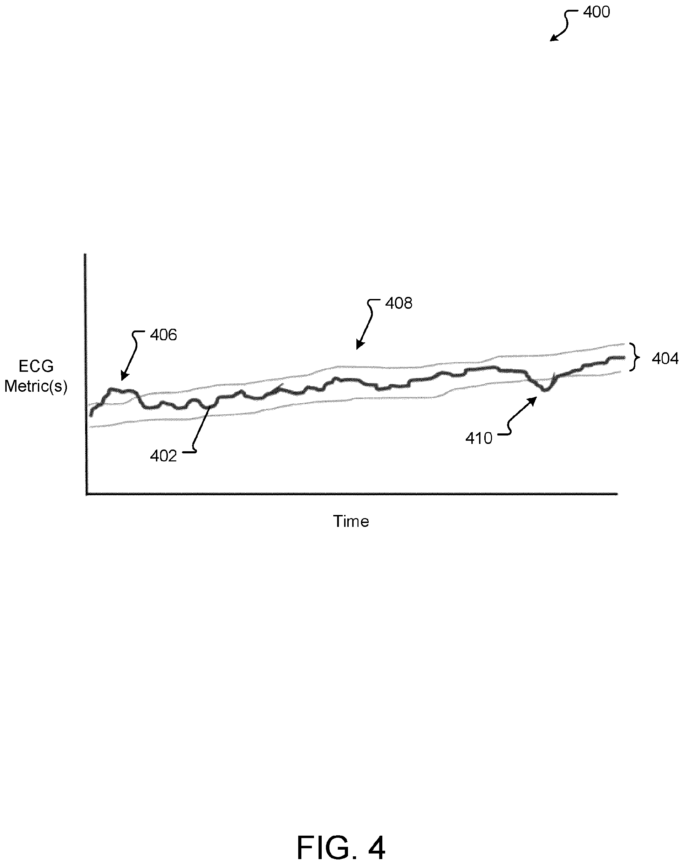

Fig. 4 is an example of long term metrics 400 which can span over the course of days, weeks, months, or years according to at least one embodiment of the present disclosure. The long term metrics 400 may be acquired by one or more aspects of Figs. 1-3. For example, the long term metrics 400 may include a plurality of cardiac measurements 402 (e.g., cardiac metrics) that are captured at a consistent rest or sleep state as described with reference to Fig. 3, where the plurality of cardiac measurements 402 are captured by components of an SCS system, such as the device 102, the leads 104, and the electrodes as described with reference to Fig. 1. The consistent cardiac measurements may result in a long term trend 404 (e.g., longitudinal trend), such that if specific cardiac measurements fall outside the long term trend 404, the SCS system can make adjustments to the stimulation to maintain or increase pain relief. In some examples, the plurality of cardiac measurements 402 may be captured across a plurality of days, weeks, months, etc., for the patient. In the example of Fig. 4, the plurality of cardiac measurements 402 may represent a plurality of HRV measurements captured for the patient, but additional cardiac measurements may be used to establish the long term trend 404.

A first time instance 406 may represent a positive response to the SCS implant (e.g., the device 102 implanted within the patient) and corresponding stimulation signal being applied to the patient. A time period 408 may represent an upward trend overall as the patient's well-being increases with the SCS treatment. At a second time instance 410, the plurality of cardiac measurements 402 may begin to change and fall outside the long term trend 404. For example, the second time instance 410 may represent a decrease in the parasympathetic metric of HRV for the patient. As a non-limiting example, if the parasympathetic HRV metric falls below 2 standard deviations of the longitudinal average for three consecutive days, this change may indicate a loss or deficiency of therapy. The change in the cardiac measurements may indicate that a change in the SCS therapy is needed to maintain or increase pain relief, such that the long term trend remains positive. The therapy adjustment may include an increase in amplitude, an adjustment of cycling, adjustment of cycling of high frequency or low frequency components independently, adjustment of charge balancing strategy (e.g., recharge adjustment), an adjustment of contacts, or a different adjustment not explicitly described herein. The closed-loop strategy could be implemented in a trial phase of SCS or after full implantation of the SCS system in the patient. Additionally, the patient and/or a physician or clinician may be alerted to the change in the cardiac measurements with or without changes in SCS stimulation.

Accordingly, as described herein, the system 100 may run an algorithm configured to determine specific states associated with a sleep-wake cycle of the patient (e.g., specific sleep states, when the patient wakes from sleep, etc.). Subsequently, the system 100 may measure one or more cardiac signals of the patient upon detecting the specific states of the sleep-wake cycle of the patient determined by the algorithm, where the one or more cardiac signals are measured via the device 102 (e.g., implantable device, implantable pulse generator, pulse generator, etc.), the leads 104, and/or the electrodes. In some embodiments, the specific states of the sleep-wake cycle when the cardiac signals are measured may include instances within a threshold amount of time after the patient falls asleep (e.g., within an hour after falling asleep), when the patient is resting before sleep, when the patient is initially waking (e.g., but is still in bed), sleep stages before waking occurs (e.g., last NREM1 stage before waking, NREM 2 stage, etc.), throughout a total duration of sleep, or a combination thereof. Additionally, the cardiac signals measured at the specific stages may include heart rate, HRV, respiration, etc. In some embodiments, the cardiac measurements may be configured to last from 30 seconds up to 10 minutes.

By measuring the cardiac signals (e.g., daily and/or nightly cardiac electrogram measurements) using the implanted system (e.g., the system 100 comprising the device 102, the leads 104, and the electrodes) at a consistent resting state (e.g., the specific states of the sleep-wake cycle as described with reference to Fig. 3), the system may establish a longitudinal trend of cardiac metrics for the patient (e.g., the long term trend 404). Subsequently, the cardiac metrics can then be used to inform and/or adjust a closed-loop therapy (e.g., via the system 100, such as a neuromodulation system, SCS system, etc.) for the patient. Additionally or alternatively, a difference in cardiac metrics collected at two different states of the sleep-wake cycle may be used to inform the closed-loop therapy. For example, the HRV at different sleep states may be compared towards closed-loop therapy.

Turning to Fig. 5, a block diagram of a system 500 according to at least one embodiment of the present disclosure is shown. The system 500 may be used to establish a longitudinal trend of a patient's cardiac metrics for informing closed-loop neuromodulation as described herein. In some examples, the system 500 may implement aspects of or may be implemented by aspects of Figs. 1-4 as described herein. For example, the system 500 may be used with a device 514, leads 516, and/or electrodes 518, and/or carry out one or more other aspects of one or more of the methods disclosed herein. The device 514 may represent an example of the device 102 or a component of the device 102 as described with reference to Fig. 1 (e.g., implantable pulse generator), where the leads 516 and the electrodes 518 may represent the leads 104 and corresponding electrodes/cuff electrodes as described with reference to Fig. 1. The system 500 comprises a computing device 502, a system 512, a database 530, and/or a cloud or other network 534. Systems according to other embodiments of the present disclosure may comprise more or fewer components than the system 500. For example, the system 500 may not include one or more components of the computing device 502, the database 530, and/or the cloud 534.

The system 512 may comprise the device 514, leads 516, and the electrodes 518. As previously described, the device 514 may be configured to generate a current (e.g., therapeutic electrical signal, stimulation signal, electrical stimulation signal, etc.), and the leads 516 and the electrodes 518 may comprise a plurality of electrodes configured to carry the current from the device 514 and apply the current to an anatomical element based on the electrodes being implanted on or near the anatomical element (e.g., stimulation target, such as the spinal cord 108 and/or nearby nerves to the spinal cord 108). In some examples, the device 514, leads 516, and electrodes 518 may be configured to measure a physiological response of the patient (e.g., prior to applying the current to the anatomical element, after the current is applied, etc.).

The system 512 may communicate with the computing device 502 to receive instructions such as instructions 524 for applying a current to the anatomical element and/or delivering the pharmacological agent to the anatomical element. The system 512 may also provide data (such as data received from an electrodes 518 capable of recording data), which may be used to optimize the electrodes 518 and/or to optimize parameters of the current generated by the device 514.

The computing device 502 comprises a processor 504, a memory 506, a communication interface 508, and a user interface 510. Computing devices according to other embodiments of the present disclosure may comprise more or fewer components than the computing device 502.

The processor 504 of the computing device 502 may be any processor described herein or any similar processor. The processor 504 may be configured to execute instructions 524 stored in the memory 506, which instructions may cause the processor 504 to carry out one or more computing steps utilizing or based on data received from the system 512, the database 530, and/or the cloud 534.

The memory 506 may be or comprise RAM, DRAM, SDRAM, other solid-state memory, any memory described herein, or any other tangible, non-transitory memory for storing computer-readable data and/or instructions. The memory 506 may store information or data useful for completing, for example, any steps of the methods 600, 700, and/or 800 described herein, or of any other methods. The memory 506 may store, for example, instructions and/or machine learning models that support one or more functions of the system 512. For instance, the memory 506 may store content (e.g., instructions and/or machine learning models) that, when executed by the processor 504, enable a cardiac metric measurement 520, a rest state determination 522, a measurement trigger 524, and a therapy determination 528.

The cardiac metric measurement 520 enables the processor 504 to measure (e.g., via the device 514, the leads 516, and/or the electrodes 518) one or more signals with cardiac activity (e.g., cardiac electrogram measurements, where different cardiac metrics, such as heart rate, HRV, respiration, etc., can be derived from the cardiac electrogram measurements) of the patient upon detecting specific states of the sleep-wake cycle of the patient. For example, as described with reference to Figs. 1-4, the cardiac signals may be measured on a daily or nightly basis during a consistent rest state and/or sleep state for the patient. The cardiac metric measurement 520 also enables the processor 504 to establish a longitudinal trend of cardiac metrics for the patient based at least in part on a plurality of signals with cardiac activity for the patient measured during the specific states of the sleep-wake cycle across a plurality of days. In some examples, the cardiac metric measurement 520 also enables the processor 504 to measure (e.g., via the device 514, the leads 516, and/or the electrodes 518) a first signal during a first specific state of the sleep-wake cycle (e.g., first sleep state) and a second signal during a second specific state of the sleep-wake cycle (e.g., second sleep state). In some embodiments, instructions stored in the memory 506 may cause the processor 504 to perform the cardiac metric measurement 520 as described.

The rest state determination 522 enables the processor 504 to determine specific states associated with a sleep-wake cycle of a patient, where the specific states are used to trigger the processor 504 to perform the cardiac metric measurement 520 described above. For example, as described with reference to Figs. 1-4, an algorithm may be used to determine the specific sleep stages of the sleep-wake cycle based on one or more input signals, such as cardiac input signals, accelerometry input signals, the time of day, respiration measurements (e.g., cardiac-derived respiration and/or respiration measurements derived, for example, from an accelerometer, impedance based measurements, optical sensors, etc.), additional information from other sensors, information from external devices (e.g., smartphone, wearables, etc.), or a combination thereof. In some examples, the specific sleep stages or states may include instances within a threshold amount of time after the patient falls asleep (e.g., within the first hour of sleep), when the patient is resting before sleep, when the patient is initially waking, sleep stages before waking occurs (e.g., NREM 1, NREM 2, etc.), throughout a total duration of sleep, or a combination thereof. In some embodiments, instructions stored in the memory 506 may cause the processor 504 to perform the rest state determination 522 as described.

The measurement trigger 524 enables the processor 504 to trigger the components of the system 512 to record the cardiac metrics upon detecting the specific states of the sleep-wake cycle of the patient. For example, as described with reference to Figs. 1-4, the system 512 may measure one or more signals with cardiac activity of the patient upon detecting the specific states of the sleep-wake cycle of the patient determined by the algorithm. The measurement trigger 524 also enables the processor 504 to configure a duration for measuring the one or more signals, where the one or more signals are measured according to the configured duration (e.g., from 30 seconds up to minutes, or a different time duration not explicitly listed herein). In some embodiments, instructions stored in the memory 506 may cause the processor 504 to perform the measurement trigger 524 as described.

The therapy determination 528 enables the processor 504 to determine one or more parameters for applying the therapeutic electrical signal to the anatomical element based at least in part on the one or more cardiac signals. In some embodiments, the therapy determination 528 may enable the processor 504 to determine one or more cardiac metrics fall outside the longitudinal trend of cardiac metrics and to adjust one or more therapy parameters for applying the therapeutic electrical signal to the anatomical element based at least in part on determining the one or more cardiac metrics fall outside the longitudinal trend of cardiac metrics. For example, as described with reference to Figs. 1-4, the one or more adjusted therapy parameters may comprise an amplitude adjustment, frequency adjustment, pulse width adjustment, adjustment of duty cycling, adjustment of cycling of high frequency or low frequency components independently, adjustment of charge balancing strategy (e.g., adjusting between active recharge, passive recharge, a combination of active and passive recharge, or any type of recharge), adjustment of selection of the plurality of electrodes, or a combination thereof.

Additionally or alternatively, the therapy determination 528 enables the processor 504 to provide the longitudinal trend of the patient's cardiac metrics and/or the daily/nightly cardiac electrogram-derived metrics (e.g., displayed via a user interface) to a physician, a clinician, an operator, the patient, or another user for tracking overall patient health (e.g., for patient monitoring). In some embodiments, the longitudinal trend of the patient's cardiac metrics and/or the daily/nightly cardiac electrogram-derived metrics may be provided to the physician, clinician, operator, patient, or other user for review and/or analysis to inform or determine therapy adjustment decisions. In some embodiments, instructions stored in the memory 506 may cause the processor 504 to perform the therapy determination 528 as described.

In some embodiments, multiple components of the system 500 may work together to perform the techniques described herein (e.g., for reporting or providing the longitudinal trend data, leveraging information or signals from additional devices, etc.). For example, the cloud 534 may leverage signals from wearables and/or other information for deriving the cardiac metrics described herein. In some embodiments, the algorithm described herein may combine information stored in the cloud 534, the database 530, or elsewhere outside the system 512 and/or outside the computing device 502 to determine the cardiac metrics. For example, the algorithm may use images (e.g., X-rays, fluoroscopy images, magnetic resonance (MR) images, etc.), medical records, etc., that are stored in the cloud 534, the database 530, or elsewhere outside the system 512 and/or outside the computing device 502 to refine and/or update algorithm parameters. Additionally, some of the therapy adjustment analysis, algorithm updates, etc., may be performed off-device (e.g., outside the system 512 and/or outside the computing device 502), in the cloud 534, etc., and the analysis, updates, etc., can then be pushed back to components of the system 512 and/or the computing device 502.

Content stored in the memory 506, if provided as in instruction, may, in some embodiments, be organized into one or more applications, modules, packages, layers, or engines. Alternatively or additionally, the memory 506 may store other types of content or data (e.g., machine learning models, artificial neural networks, deep neural networks, etc.) that can be processed by the processor 504 to carry out the various method and features described herein. Thus, although various contents of memory 506 may be described as instructions, it should be appreciated that functionality described herein can be achieved through use of instructions, algorithms, and/or machine learning models. The data, algorithms, and/or instructions may cause the processor 504 to manipulate data stored in the memory 506 and/or received from or via the system 512, the database 530, and/or the cloud 534.

The computing device 502 may also comprise a communication interface 508. The communication interface 508 may be used for receiving data (for example, data from the electrodes 518 capable of recording data) or other information from an external source (such as the system 512, the database 530, the cloud 534, and/or any other system or component not part of the system 500), and/or for transmitting instructions, images, or other information to an external system or device (e.g., another computing device 502, the system 512, the database 530, the cloud 534, and/or any other system or component not part of the system 500). The communication interface 508 may comprise one or more wired interfaces (e.g., a USB port, an Ethernet port, a Firewire port) and/or one or more wireless transceivers or interfaces (configured, for example, to transmit and/or receive information via one or more wireless communication protocols such as 802.1 1a/b/g/n, Bluetooth, NFC, ZigBee, and so forth). In some embodiments, the communication interface 508 may be useful for enabling the device 502 to communicate with one or more other processors 504 or computing devices 502, whether to reduce the time needed to accomplish a computing-intensive task or for any other reason.

The computing device 502 may also comprise one or more user interfaces 510. The user interface 510 may be or comprise a keyboard, mouse, trackball, monitor, television, screen, touchscreen, and/or any other device for receiving information from a user and/or for providing information to a user. The user interface 510 may be used, for example, to receive a user selection or other user input regarding any step of any method described herein. Notwithstanding the foregoing, any required input for any step of any method described herein may be generated automatically by the system 500 (e.g., by the processor 504 or another component of the system 500) or received by the system 500 from a source external to the system 500. In some embodiments, the user interface 510 may be useful to allow a surgeon or other user to modify instructions to be executed by the processor 504 according to one or more embodiments of the present disclosure, and/or to modify or adjust a setting of other information displayed on the user interface 510 or corresponding thereto.

Although the user interface 510 is shown as part of the computing device 502, in some embodiments, the computing device 502 may utilize a user interface 510 that is housed separately from one or more remaining components of the computing device 502. In some embodiments, the user interface 510 may be located proximate one or more other components of the computing device 502, while in other embodiments, the user interface 510 may be located remotely from one or more other components of the computer device 502.

Though not shown, the system 500 may include a controller, though in some embodiments the system 500 may not include the controller. The controller may be an electronic, a mechanical, or an electro-mechanical controller. The controller may comprise or may be any processor described herein. The controller may comprise a memory storing instructions for executing any of the functions or methods described herein as being carried out by the controller. In some embodiments, the controller may be configured to simply convert signals received from the computing device 502 (e.g., via a communication interface 105) into commands for operating the system 512 (and more specifically, for actuating the device 514 and/or the electrodes 518). In other embodiments, the controller may be configured to process and/or convert signals received from the system 512. Further, the controller may receive signals from one or more sources (e.g., the system 512) and may output signals to one or more sources.

The database 530 may store information such as patient data, results of a stimulation and/or neuromodulation procedure, stimulation and/or neuromodulation parameters, current parameters, electrode parameters, etc. The database 530 may be configured to provide any such information to the computing device 502 or to any other device of the system 500 or external to the system 500, whether directly or via the cloud 534. In some embodiments, the database 530 may be or comprise part of a hospital image storage system, such as a picture archiving and communication system (PACS), a health information system (HIS), and/or another system for collecting, storing, managing, and/or transmitting electronic medical records.

The cloud 534 may be or represent the Internet or any other wide area network. The computing device 502 may be connected to the cloud 534 via the communication interface 508, using a wired connection, a wireless connection, or both. In some embodiments, the computing device 502 may communicate with the database 530 and/or an external device (e.g., a computing device) via the cloud 534.

The system 500 or similar systems may be used, for example, to carry out one or more aspects of any of the methods 600, 700, and/or 800 as described herein. The system 500 or similar systems may also be used for other purposes.

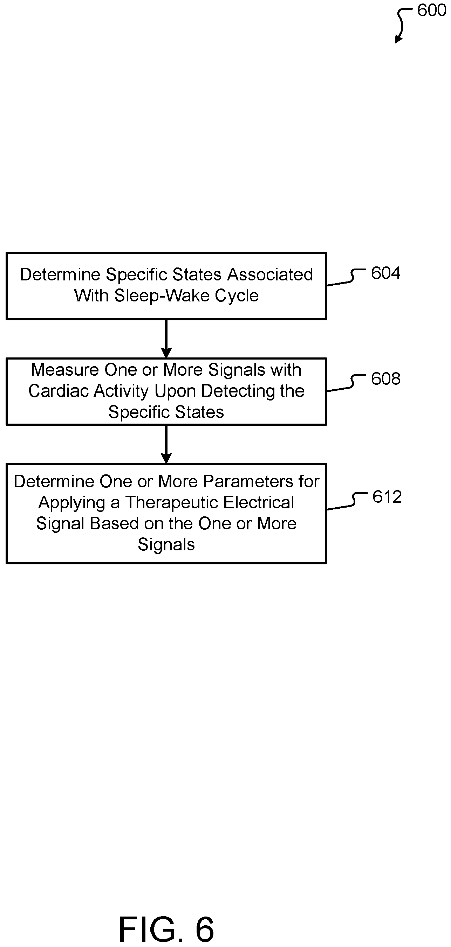

Fig. 6 depicts a method 600 that may be used, for example, to determine parameters for a closed-loop neuromodulation system based on cardiac metrics measured during consistent rest states for a patient over a period of time.

The method 600 (and/or one or more steps thereof) may be carried out or otherwise performed, for example, by at least one processor. The at least one processor may be the same as or similar to the processor(s) 504 of the computing device 502 described above. The at least one processor may be the same as or similar to the processor(s) of the device 104, 514 described above. The at least one processor may be part of the device 104, 514 (such as an implantable pulse generator) or part of a control unit in communication with the device 104, 514. A processor other than any processor described herein may also be used to execute the method 600. The at least one processor may perform the method 600 by executing elements stored in a memory such as the memory 506. The elements stored in the memory and executed by the processor may cause the processor to execute one or more steps of a function as shown in method 600. One or more portions of a method 600 may be performed by the processor executing any of the contents of memory, such as a cardiac metric measurement 520, a rest state determination 522, a measurement trigger 524, and/or a therapy determination 528.

The method 600 comprises determining specific states associated with a sleep-wake cycle of a patient (step 604). For example, the specific states of the sleep-wake cycle are detected based at least in part on an algorithm as described with reference to Figs. 1-5, where the algorithm uses accelerometry measurements, cardiac metrics, a time of day, respiration measurements, additional information from external devices, or a combination thereof, as input parameters to detect the specific states of the sleep-wake cycle. In some embodiments, the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof. Additionally, the specific states associated with the sleep-wake cycle of the patient may be determined based on known methods for sleep classification.

The method 600 also comprises measuring, via one or more of a plurality of electrodes (e.g., and/or the device 104, 514), one or more signals with cardiac activity of the patient upon detecting the specific states of the sleep-wake cycle of the patient (step 608). That is, signals with cardiac activity may be collected during specific sleep/wake states that have been determined (e.g., based on known methods for sleep classification), where different cardiac metrics can be derived from the collected signals. In some embodiments, a duration may be configured for measuring the one or more cardiac signals, where the one or more cardiac signals are measured according to the configured duration (e.g., for anywhere from 30 seconds up to 10 minutes, or a different duration). Additionally, the cardiac signals may comprise cardiac electrogram measurements, a heart rate, an HRV, respiration, or a combination thereof, for the patient.

In some embodiments, the measuring may be automatic. In other embodiments, the measuring start and/or end based on user input. For example, a patient may request to perform cardiac measuring or recording at specific times and/or for specific durations. The system may also prompt the user (through, for example, a communication interface such as the communication interface 508) to establish a state such as, for example, a resting position, a relaxed position, and/or a stable position to record or measure. The system may also prompt the user to begin cardiac recording or measurement. The user can then begin or delay the cardiac recording or measurement until a desired time. Information obtained from the measurements or recordings can be used for reporting a general wellbeing of the patient, can be used to store templates of the patient, and/or be used to aid in data processing.

The recording or measuring (whether based on patient input, a prompt to the patient, or automatically) may be useful during, for example, calibration or adjustment of a system such as the system 512. For example, the patient may determine that the system should be recalibrated or adjusted and the patient can then request or cause the system to perform the cardiac measuring or recording. The system can then use the current recording or measurements to adjust or calibrate the system (or a device such as the device 514 of the system).

The method 600 also comprises determining one or more parameters for applying a therapeutic electrical signal to an anatomical element of the patient (e.g., spinal cord and/or nearby nerves to the spinal cord) based at least in part on the one or more cardiac signals (step 612). For example, an amplitude of the therapeutic electrical signal, a duty cycle for the therapeutic electrical signal, a cycling of high frequency or low frequency components independently, charge balancing strategy (e.g., recharge adjustments), which electrodes deliver the therapeutic electrical signal, or additional parameters may be determined based on the one or more cardiac signals (e.g., indicating how the patient responds to the therapeutic electrical signal being applied).

The present disclosure encompasses embodiments of the method 600 that comprise more or fewer steps than those described above, and/or one or more steps that are different than the steps described above.

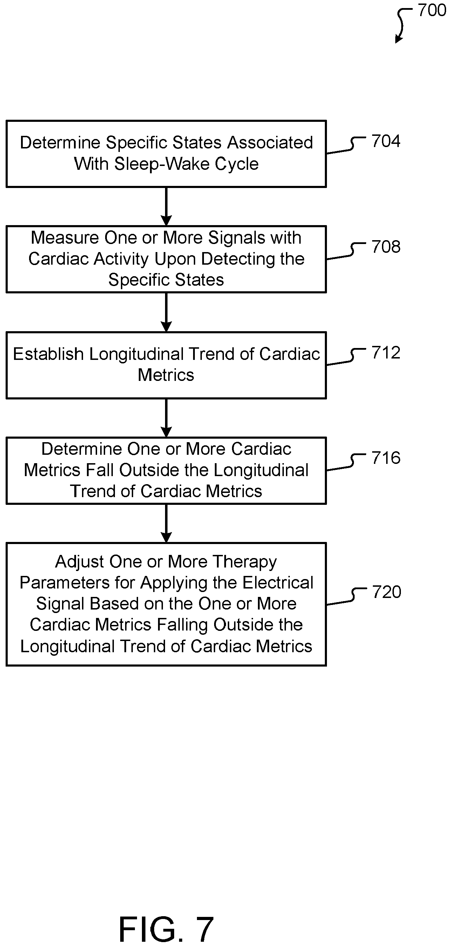

Fig. 7 depicts a method 700 that may be used, for example, to establish long term trends of cardiac measurements for a patient to guide or inform closed-loop neuromodulation.

The method 700 (and/or one or more steps thereof) may be carried out or otherwise performed, for example, by at least one processor. The at least one processor may be the same as or similar to the processor(s) 504 of the computing device 502 described above. The at least one processor may be the same as or similar to the processor(s) of the device 104, 514 described above. The at least one processor may be part of the device 104, 514 (such as an implantable pulse generator) or part of a control unit in communication with the device 104, 514. A processor other than any processor described herein may also be used to execute the method 700. The at least one processor may perform the method 700 by executing elements stored in a memory such as the memory 506. The elements stored in memory and executed by the processor may cause the processor to execute one or more steps of a function as shown in method 700. One or more portions of a method 700 may be performed by the processor executing any of the contents of memory, such as a cardiac metric measurement 520, a rest state determination 522, a measurement trigger 524, and/or a therapy determination 528.

The method 700 comprises determining specific states associated with a sleep-wake cycle of a patient (step 704). Step 704 may implement similar aspect of step 604 as described with reference to Fig. 6. The method 700 also comprises measuring, via one or more of a plurality of electrodes (e.g., and/or the device 104, 514), one or more cardiac signals of the patient upon detecting the specific states of the sleep-wake cycle of the patient (step 708). Step 708 may implement similar aspect of step 608 as described with reference to Fig. 6.

The method 700 also comprises establishing a longitudinal trend of cardiac metrics for the patient based at least in part on a plurality of cardiac signals for the patient measured during the specific states of the sleep-wake cycle across a plurality of days (step 712). For example, the plurality of cardiac signal may be measured during a consistent rest state for the patient.

The method 700 also comprises determining the one or more cardiac signals fall outside the longitudinal trend of cardiac metrics (step 716). The method 700 also comprises adjusting one or more therapy parameters for applying the therapeutic electrical signal to the anatomical element based at least in part on determining the one or more cardiac signals fall outside the longitudinal trend of cardiac metrics (step 720). For example, the one or more adjusted therapy parameters comprise an amplitude adjustment, frequency adjustment, pulse width adjustment, adjustment of duty cycling, adjustment of cycling of high frequency or low frequency components independently, adjustment of charge balancing strategy, adjustment of selection of the plurality of electrodes, or a combination thereof.

Additionally or alternatively, the longitudinal trend of cardiac metrics for the patient may be output (e.g., via a user interface), and one or more parameters for applying the therapeutic electrical signal to the anatomical element may be determined based at least in part on outputting the longitudinal trend of cardiac metrics for the patient.

The present disclosure encompasses embodiments of the method 700 that comprise more or fewer steps than those described above, and/or one or more steps that are different than the steps described above.

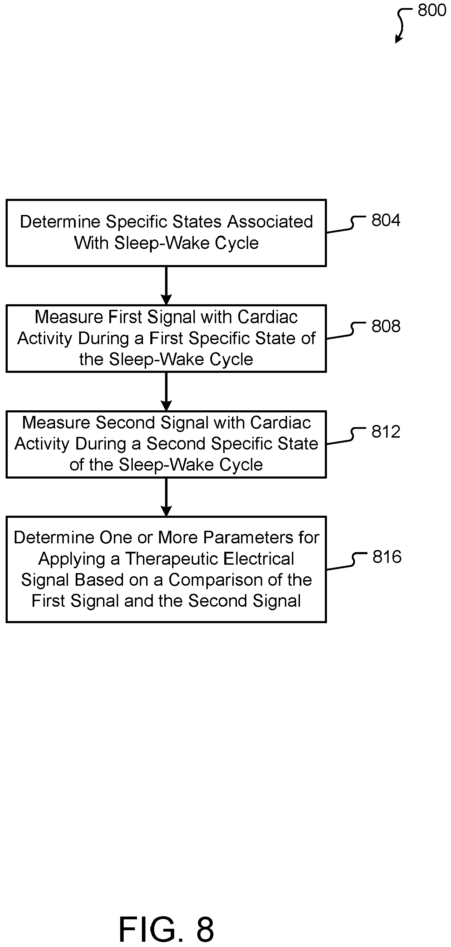

Fig. 8 depicts a method 800 that may be used, for example, to use a difference in cardiac metrics (e.g., cardiac electrogram-derived metrics) collected at two different states to inform closed-loop therapy.

The method 800 (and/or one or more steps thereof) may be carried out or otherwise performed, for example, by at least one processor. The at least one processor may be the same as or similar to the processor(s) 504 of the computing device 502 described above. The at least one processor may be the same as or similar to the processor(s) of the device 104, 514 described above. The at least one processor may be part of the device 104, 514 (such as an implantable pulse generator) or part of a control unit in communication with the device 104, 514. A processor other than any processor described herein may also be used to execute the method 800. The at least one processor may perform the method 800 by executing elements stored in a memory such as the memory 506. The elements stored in memory and executed by the processor may cause the processor to execute one or more steps of a function as shown in method 800. One or more portions of a method 800 may be performed by the processor executing any of the contents of memory, such as a cardiac metric measurement 520, a rest state determination 522, a measurement trigger 524, and/or a therapy determination 528.

The method 800 comprises determining specific states associated with a sleep-wake cycle of a patient (step 804). Step 804 may implement similar aspect of steps 604 and 704 as described with reference to Figs. 6 and 7.

The method 800 also comprises measuring, via one or more of a plurality of electrodes (e.g., and/or the device 104, 514), a first cardiac signal during a first specific state of the sleep-wake cycle (step 808). For example, the first specific state may comprise a first sleep state for the patient.

The method 800 also comprises measuring, via one or more of the plurality of electrodes (e.g., and/or the device 104, 514), a second cardiac signal during a second specific state of the sleep-wake cycle (step 812). For example, the second specific state may comprise a second sleep state for the patient.

The method 800 also comprises determine one or more parameters for applying the therapeutic electrical signal to the anatomical element based at least in part on a comparison of the first cardiac signal and the second cardiac signal (step 816).

The present disclosure encompasses embodiments of the method 800 that comprise more or fewer steps than those described above, and/or one or more steps that are different than the steps described above.

As noted above, the present disclosure encompasses methods with fewer than all of the steps identified in Figs. 6, 7, and 8 (and the corresponding description of the methods 600, 700, and 800), as well as methods that include additional steps beyond those identified in Figs. 6, 7, and 8 (and the corresponding description of the methods 600, 700, and 800). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the foregoing has included description of one or more aspects, embodiments, and/or configurations and certain variations and modifications, other variations, combinations, and modifications are within the scope of the disclosure, e.g., as may be within the skill and knowledge of those in the art, after understanding the present disclosure. It is intended to obtain rights which include alternative aspects, embodiments, and/or configurations to the extent permitted, including alternate, interchangeable and/or equivalent structures, functions, ranges or steps to those claimed, whether or not such alternate, interchangeable and/or equivalent structures, functions, ranges or steps are disclosed herein, and without intending to publicly dedicate any patentable subject matter.

Further disclosed herein is the subject-matter of the following clauses:
1. A system for informing a therapeutic procedure, comprising:
   a pulse generator configured to generate a therapeutic electrical signal;
   one or more leads in communication with the pulse generator and configured to transmit the electrical signal to a plurality of electrodes;
   the plurality of electrodes in communication with the one or more leads, the plurality of electrodes configured to apply the electrical signal to an anatomical element of a patient and configured to measure a physiological response;
   a processor; and
   a memory storing data for processing by the processor, the data, when processed, causes the processor to:
      determine specific states associated with a sleep-wake cycle of the patient;
      measure, via one or more of the plurality of electrodes, one or more signals with cardiac activity of the patient upon detecting the specific states of the sleep-wake cycle of the patient; and
      determine one or more parameters for applying the therapeutic electrical signal to the anatomical element based at least in part on the one or more signals with cardiac activity.
2. The system of clause 1, wherein the memory stores further data for processing by the processor that, when processed, causes the processor to:
   establish a longitudinal trend of cardiac metrics for the patient based at least in part on a plurality of signals with cardiac activity for the patient measured during the specific states of the sleep-wake cycle across a plurality of days.
3. The system of clause 2, wherein the memory stores further data for processing by the processor that, when processed, causes the processor to:
   determine one or more cardiac metrics fall outside the longitudinal trend of cardiac metrics; and
   adjust one or more therapy parameters for applying the therapeutic electrical signal to the anatomical element based at least in part on determining the one or more cardiac metrics fall outside the longitudinal trend of cardiac metrics, or
   provide, via a user interface, an alert indicating that the one or more cardiac metrics fall outside the longitudinal trend of cardiac metrics.
4. The system of clause 3, wherein the one or more adjusted therapy parameters comprise an amplitude adjustment, adjustment of duty cycling, adjustment of cycling of high frequency or low frequency components independently, adjustment of frequency, adjustment of pulse width, adjustment of charge balancing strategy, adjustment of selection of the plurality of electrodes, or a combination thereof.
5. The system of clause 2, wherein the memory stores further data for processing by the processor that, when processed, causes the processor to:
   output, via a user interface, the longitudinal trend of cardiac metrics for the patient, wherein the one or more parameters for applying the therapeutic electrical signal to the anatomical element are determined based at least in part on outputting the longitudinal trend of cardiac metrics for the patient.
6. The system of clause 1 or of any of the preceding clauses, wherein the data stored in the memory that, when processed causes the processor to measure the one or more signals with cardiac activity further causes the system to:
   measure, via one or more of the plurality of electrodes, a first signal with cardiac activity during a first specific state of the sleep-wake cycle; and
   measure, via one or more of the plurality of electrodes, a second signal with cardiac activity during a second specific state of the sleep-wake cycle, wherein the one or more parameters for applying the therapeutic electrical signal to the anatomical element are determined based at least in part on a comparison of the first signal and the second signal.
7. The system of clause 6, wherein the first specific state comprises a first sleep state for the patient, and the second specific state comprises a second sleep state for the patient.
8. The system of clause 1 or of any of the preceding clauses, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.
9. The system of clause 1 or of any of the preceding clauses, wherein the memory stores further data for processing by the processor that, when processed, causes the processor to:
   configure a duration for measuring the one or more signals with cardiac activity, wherein the one or more signals with cardiac activity are measured according to the configured duration.
10. The system of clause 1 or of any of the preceding clauses, wherein the specific states of the sleep-wake cycle are detected based at least in part on an algorithm that uses accelerometry measurements, cardiac metrics, a time of day, respiration measurements, additional information from external devices, or a combination thereof, as input parameters.
11. The system of clause 1 or of any of the preceding clauses, wherein the signals with cardiac activity comprise cardiac electrogram measurements, and wherein one or more cardiac metrics are derived from the cardiac electrogram measurements, the one or more cardiac metrics comprising a heart rate, a heart rate variability, respiration, or a combination thereof, for the patient.
12. A system for informing a therapeutic procedure, comprising:
   a processor; and
   a memory storing data for processing by the processor, the data, when processed, causes the processor to:
      determine specific states associated with a sleep-wake cycle of a patient;
      measure one or more signals with cardiac activity of the patient upon detecting the specific states of the sleep-wake cycle of the patient; and
      determine one or more parameters for applying a therapeutic electrical signal to an anatomical element of the patient based at least in part on the one or more signals with cardiac activity.
13. The system of clause 12, wherein the memory stores further data for processing by the processor that, when processed, causes the processor to:
   establish a longitudinal trend of cardiac metrics for the patient based at least in part on a plurality of signals with cardiac activity for the patient measured during the specific states of the sleep-wake cycle across a plurality of days.
14. The system of clause 13, wherein the memory stores further data for processing by the processor that, when processed, causes the processor to:
   determine one or more cardiac metrics fall outside the longitudinal trend of cardiac metrics; and
   adjust one or more therapy parameters for applying the therapeutic electrical signal to the anatomical element based at least in part on determining the one or more cardiac metrics fall outside the longitudinal trend of cardiac metrics, or
   provide, via a user interface, an alert indicating that the one or more cardiac metrics fall outside the longitudinal trend of cardiac metrics.
15. The system of clause 14, wherein the one or more adjusted therapy parameters comprise an amplitude adjustment, adjustment of duty cycling, adjustment of frequency, adjustment of pulse width, adjustment of cycling of high frequency or low frequency components independently, adjustment of charge balancing strategy, adjustment of selection of the plurality of electrodes, or a combination thereof.
16. The system of clause 12 or of any of clauses 12-15, wherein the data stored in the memory that, when processed causes the processor to measure the one or more signals with cardiac activity further causes the system to:
   measure a first signal with cardiac activity during a first specific state of the sleep-wake cycle; and
   measure a second signal with cardiac activity during a second specific state of the sleep-wake cycle, wherein the one or more parameters for applying the therapeutic electrical signal to the anatomical element are determined based at least in part on a comparison of the first signal and the second signal.
17. The system of clause 12 or of any of clauses 12-16, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.
18. The system of clause 11 or 12 or of any of clauses 11-18, wherein the specific states of the sleep-wake cycle are detected based at least in part on an algorithm that uses accelerometry measurements, cardiac metrics, a time of day, respiration measurements, additional information from external devices, or a combination thereof, as input parameters.
19. A system for informing a therapeutic procedure, comprising:
   a pulse generator configured to generate a therapeutic electrical signal;
   one or more leads in communication with the pulse generator and configured to transmit the therapeutic electrical signal to a plurality of electrodes; and
   the plurality of electrodes in communication with the one or more leads, the plurality of electrodes configured to apply the therapeutic electrical signal to an anatomical element of a patient and configured to measure a physiological response,
   wherein the therapeutic electrical signal is applied to the anatomical element based at least in part on one or more cardiac metrics derived from one or more signals with cardiac activity measured via one or more of the plurality of electrodes at specific states of a sleep-wake cycle of the patient.
20. The system of clause 19, wherein the specific states of the sleep-wake cycle comprise states within a threshold amount of time after the patient falls asleep, when the patient is resting before sleep, when the patient is initially waking, sleep stages before waking occurs, or a combination thereof.

## Claims

1. A system for informing a therapeutic procedure, comprising:
a pulse generator configured to generate a therapeutic electrical signal;
one or more leads in communication with the pulse generator and configured to transmit the electrical signal to a plurality of electrodes;
the plurality of electrodes in communication with the one or more leads, the plurality of electrodes configured to apply the electrical signal to an anatomical element of a patient and configured to measure a physiological response;
a processor; and
a memory storing data for processing by the processor, the data, when processed, causes the processor to:
determine specific states associated with a sleep-wake cycle of the patient;
measure, via one or more of the plurality of electrodes, one or more signals with cardiac activity of the patient upon detecting the specific states of the sleep-wake cycle of the patient; and
determine one or more parameters for applying the therapeutic electrical signal to the anatomical element based at least in part on the one or more signals with cardiac activity.

2. The system of claim 1, wherein the memory stores further data for processing by the processor that, when processed, causes the processor to:
establish a longitudinal trend of cardiac metrics for the patient based at least in part on a plurality of signals with cardiac activity for the patient measured during the specific states of the sleep-wake cycle across a plurality of days.

3. The system of claim 2, wherein the memory stores further data for processing by the processor that, when processed, causes the processor to:
determine one or more cardiac metrics fall outside the longitudinal trend of cardiac metrics; and
adjust one or more therapy parameters for applying the therapeutic electrical signal to the anatomical element based at least in part on determining the one or more cardiac metrics fall outside the longitudinal trend of cardiac metrics, or
provide, via a user interface, an alert indicating that the one or more cardiac metrics fall outside the longitudinal trend of cardiac metrics.

4. The system of claim 3, wherein the one or more adjusted therapy parameters comprise an amplitude adjustment, adjustment of duty cycling, adjustment of cycling of high frequency or low frequency components independently, adjustment of frequency, adjustment of pulse width, adjustment of charge balancing strategy, adjustment of selection of the plurality of electrodes, or a combination thereof.

5. The system of claim 2, wherein the memory stores further data for processing by the processor that, when processed, causes the processor to:
output, via a user interface, the longitudinal trend of cardiac metrics for the patient, wherein the one or more parameters for applying the therapeutic electrical signal to the anatomical element are determined based at least in part on outputting the longitudinal trend of cardiac metrics for the patient.

6. The system of any of the preceding claims, wherein the data stored in the memory that, when processed causes the processor to measure the one or more signals with cardiac activity further causes the system to:
measure, via one or more of the plurality of electrodes, a first signal with cardiac activity during a first specific state of the sleep-wake cycle; and
measure, via one or more of the plurality of electrodes, a second signal with cardiac activity during a second specific state of the sleep-wake cycle, wherein the one or more parameters for applying the therapeutic electrical signal to the anatomical element are determined based at least in part on a comparison of the first signal and the second signal, wherein optionally the first specific state comprises a first sleep state for the patient, and the second specific state comprises a second sleep state for the patient.

7. The system of any of the preceding claims, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof.

8. The system of any of the preceding claims, wherein the memory stores further data for processing by the processor that, when processed, causes the processor to:
configure a duration for measuring the one or more signals with cardiac activity, wherein the one or more signals with cardiac activity are measured according to the configured duration.

9. The system of any of the preceding claims, wherein the specific states of the sleep-wake cycle are detected based at least in part on an algorithm that uses accelerometry measurements, cardiac metrics, a time of day, respiration measurements, additional information from external devices, or a combination thereof, as input parameters, and/or wherein the signals with cardiac activity comprise cardiac electrogram measurements, and wherein one or more cardiac metrics are derived from the cardiac electrogram measurements, the one or more cardiac metrics comprising a heart rate, a heart rate variability, respiration, or a combination thereof, for the patient.

10. A system for informing a therapeutic procedure, comprising:
a processor; and
a memory storing data for processing by the processor, the data, when processed, causes the processor to:
determine specific states associated with a sleep-wake cycle of a patient;
measure one or more signals with cardiac activity of the patient upon detecting the specific states of the sleep-wake cycle of the patient; and
determine one or more parameters for applying a therapeutic electrical signal to an anatomical element of the patient based at least in part on the one or more signals with cardiac activity.

11. The system of claim 10, wherein the memory stores further data for processing by the processor that, when processed, causes the processor to:
establish a longitudinal trend of cardiac metrics for the patient based at least in part on a plurality of signals with cardiac activity for the patient measured during the specific states of the sleep-wake cycle across a plurality of days.

12. The system of claim 11, wherein the memory stores further data for processing by the processor that, when processed, causes the processor to:
determine one or more cardiac metrics fall outside the longitudinal trend of cardiac metrics; and
adjust one or more therapy parameters for applying the therapeutic electrical signal to the anatomical element based at least in part on determining the one or more cardiac metrics fall outside the longitudinal trend of cardiac metrics, or
provide, via a user interface, an alert indicating that the one or more cardiac metrics fall outside the longitudinal trend of cardiac metrics, wherein optionally the one or more adjusted therapy parameters comprise an amplitude adjustment, adjustment of duty cycling, adjustment of frequency, adjustment of pulse width, adjustment of cycling of high frequency or low frequency components independently, adjustment of charge balancing strategy, adjustment of selection of the plurality of electrodes, or a combination thereof.

13. The system of any of claims 10-12, wherein the data stored in the memory that, when processed causes the processor to measure the one or more signals with cardiac activity further causes the system to:
measure a first signal with cardiac activity during a first specific state of the sleep-wake cycle; and
measure a second signal with cardiac activity during a second specific state of the sleep-wake cycle, wherein the one or more parameters for applying the therapeutic electrical signal to the anatomical element are determined based at least in part on a comparison of the first signal and the second signal.

14. The system of any of claims 10-13, wherein the specific states of the sleep-wake cycle comprise states within a threshold amount of time after the patient falls asleep, when the patient is resting before sleep, when the patient is initially waking, sleep stages before waking occurs, throughout a total duration of sleep, or a combination thereof, and/or wherein the specific states of the sleep-wake cycle are detected based at least in part on an algorithm that uses accelerometry measurements, cardiac metrics, a time of day, respiration measurements, additional information from external devices, or a combination thereof, as input parameters.

15. A system for informing a therapeutic procedure, comprising:
a pulse generator configured to generate a therapeutic electrical signal;
one or more leads in communication with the pulse generator and configured to transmit the therapeutic electrical signal to a plurality of electrodes; and
the plurality of electrodes in communication with the one or more leads, the plurality of electrodes configured to apply the therapeutic electrical signal to an anatomical element of a patient and configured to measure a physiological response,
wherein the therapeutic electrical signal is applied to the anatomical element based at least in part on one or more cardiac metrics derived from one or more signals with cardiac activity measured via one or more of the plurality of electrodes at specific states of a sleep-wake cycle of the patient, wherein optionally the specific states of the sleep-wake cycle comprise states within a threshold amount of time after the patient falls asleep, when the patient is resting before sleep, when the patient is initially waking, sleep stages before waking occurs, or a combination thereof.
